Europäisches Patentamt

European Patent Office

Office européen des brevets

(11) Publication number: **0 071 486**
**B1**

(12) **EUROPEAN PATENT SPECIFICATION**

(45) Date of publication of patent specification: **20.09.89**

(21) Application number: **82304040.7**

(22) Date of filing: **30.07.82**

(51) Int. Cl.⁴: **C 12 N 15/00**, C 12 P 21/02, C 12 N 1/20, C 07 H 21/04 // C12R1/19

(54) **Novel microorganisms of the genus Eschericia, hybrid DNA for use in their production and the use of the microorganisms in the preparation of glutathione.**

(30) Priority: **30.07.81 JP 120546/81**

(43) Date of publication of application:
**09.02.83 Bulletin 83/06**

(45) Publication of the grant of the patent:
**20.09.89 Bulletin 89/38**

(84) Designated Contracting States:
**DE FR GB IT**

(56) References cited:
**PATENTS ABSTRACTS OF JAPAN, vol. 5, no. 152 (C-73) 824r, 25th September 1981 & JA - JA - 56820**
**CHEMICAL ABSTRACTS, vol. 94, no. 13, 30th March 1981, page 405, no. 99451y, Columbus, Ohio, US. K. MURATA et al.: "Excretion of glutathione by methylglyoxal-resistant Escherichia coli"**
**CHEMICAL ABSTRACTS, vol. 83, no. 11, 15th September 1975, page 231, no. 92926w, Columbus, Ohio, US. P. APONTOWEIL et al.: "Glutathione biosynthesis in Escherichia coli K12. Properties of the enyzmes and regulation"**

(73) Proprietor: **Kimura, Akira**
**80-2, Wakamiya-dori 6-jo-agaru Kami-wakamiya-cho**
**Shimogyo-ku Kyoto-shi Kyoto-fu (JP)**

(73) Proprietor: **Murata, Kousaku**
**3-35, Nishino Hitsugawa-cho Yamashina-ku Kyoto-shi Kyoto-fu (JP)**

(72) Inventor: **Kimura, Akira**
**80-2, Wakamiya-dori 6-jo-agaru Kami-wakamiya-cho**
**Shimogyo-ku Kyoto-shi Kyoto-fu (JP)**
Inventor: **Murata, Kousaku**
**3-35, Nishino Hitsugawa-cho Yamashina-ku Kyoto-shi Kyoto-fu (JP)**

(74) Representative: **Watkins, Arnold Jack et al**
**European Patent Attorney Frank B. Dehn & Co.**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

The present invention relates to novel microorganisms of the genus *Escherichia,* especially *Escherichia coli,* hybrid DNA for use in their production and the use of the microorganisms in the preparation of glutathione.

Glutathione is a tripeptide consisting of the amino acids L-glutamic acid, L-cysteine and glycine and otherwise known as γ-L-glutamyl-L-cysteinylglycine. Glutathione is used *inter alia* as a medicament in the treatment of liver disease, as an antidote and as a biochemical reagent. Conventionally, glutathione is prepared, for example, by extraction from the microbial cells of yeast, by contacting a dried yeast having a high membrane permeability with a substrate solution containing L-glutamic acid, L-cysteine and glycine, or by contacting the microbial cells of yeast or coliform bacilli with the above-mentioned substrate solution. The preparation of the glutathione, on an industrial scale, by such known processes however commonly results in a poor yield.

The present invention is based on the discovery that glutathione may be obtained in large amount by culturing a novel microorganism derived from a bacterium of the genus *Escherichia* and/or by reacting L-glutamic acid, L-cysteine and glycine in the presence of an enzyme system obtained by culturing the novel microorganism. The production of large amounts of glutathione by a mutant strain, derived from a wild-type strain of *E. coli,* growing on a medium containing methylglyoxal (MG) has been previously demonstrated (J. G. M. *120,* 545—547, 1980 and Patent Abstracts of Japan, *5* (152), C-73 (824), 25th Sept. 1981). The present invention is also based on the discovery of novel microorganisms which, unlike the mutant disclosed in the prior art, are cysteine-requiring *and* resistant to MG and 8-hydroxyquinoline.

Thus according to one feature of the present invention there is provided a new mutant strain of *Escherichia,* which synthesises γ-glutamyl-L-cysteine synthetase and glutathione synthetase required for the synthesis of glutathione from L-glutamic acid, L-cysteine and glycine, the first said enzyme having a reduced or absent sensitivity to inhibition by glutathione and in which the introduction of the chromosomal DNA coding for said enzyme has been effected by transformation with a vector containing the said chromosomal DNA from a strain of *E. coli* having a reduced or absent sensitivity to inhibition by glutathione.

The integration of the said chromosome DNA is preferably effected by the introduction of a plasmid which is a vector of the said chromosome DNA. The plasmid is preferably a pBR322 plasmid.

The microorganism of the present invention is preferably of the species *Escherichia coli* especially *Escherichia coli* RC 912p (FERM-BP No. 48).

It is convenient to prepare a microorganism in which the strain having a reduced or absent sensitivity to inhibition by glutathione and/or the parent is *Escherichia coli* RC 912p (FERM-BP No. 47). This microorganism is described and claimed in our EP—A—71,485.

The above-mentioned microorganisms are deposited with the Fermentation Research Institute and may be obtained from this Institute.

The microorganisms of the present invention may for example be prepared by integrating chromosome DNA of a foreign strain of a bacterium of the genus *Escherichia,* preferably *Escherichia coli,* into a parent strain of the genus *Escherichia* which synthesises γ-glutamyl-L-cysteine synthetase and glutathione synthetase required to produce glutathione but wherein the γ-glutamyl-L-cysteine synthetase has a reduced or absent sensitivity to inhibition by glutathione, the foreign strain also synthesising γ-glutamyl-L-cysteine synthetase and glutathione synthetase but said γ-glutamyl-L-cysteine synthetase having a reduced or absent sensitivity to inhibition by glutathione. The parent strain may for example be deficient in the production of γ-glutamyl-L-cysteine synthetase.

The mutant strain *E. coli* RC912p (FERM-BP No. 48) may for example be obtained by a process comprising the following steps:—

(1) Preparation of a strain of *E. coli* having a reduced or absent sensitivity to inhibition by glutathione:

A wild strain of *E. coli* such as *E. coli* B 355 (ATCC 23226, J. Appl. Biochem. 1,283 (1979)), which synthesises γ-glutamyl-L-cysteine synthetase (E.C. 6.3.2.2., hereinafter referred to as GSH-I) and glutathione synthetase (E.C. 6.3.2.3., hereinafter referred to as GSH-II), is treated to induce a cysteine-requiring strain and a methylglyoxal-resistant strain. The induction of the mutant strains may be effected in conventional manner, for example, by using N-methyl-N'-nitro-N-nitrosoguanidine (hereinbefore also referred to as NTG), although it is also possible to use other mutagens such as other chemical mutagens or mutagenic radiation such as irradiation by ultraviolet light is desired. The treated strain is then cultured using a minimum medium containing L-cysteine (for example $2 \times 10^{-5}$M) and having a composition of for example $K_2HPO_4$ (0.7%), $KH_2PO_4$ (0.3%), $(NH_4)_2SO_4$ (0.1%), $MgSO_4 \cdot 7H_2O$ (0.01%) and glucose (0.5%) [pH about 7.0; hereinafter referred to as DM medium] conveniently at a temperature of 30—37°C for 16—40 hours with shaking to obtain smaller colonies, from which a cysteine-requiring strain may be obtained and cultured pure under the same or similar conditions. Separately, the strain after induction treatment is cultured under similar conditions using a DM medium containing methylglyoxal (for example $2 \times 10^{-3}$M) to obtain larger colonies from which a methylglyoxal-resistant strain may be obtained. The methylglyoxal-resistant strain thus obtained is then transferred to the above-mentioned DM medium containing the cysteine-requiring strain for culturing under the same or similar conditions. In contrast to the majority of MG-resistant cells, some colonies do not produce or excrete glutathione and thus a mutant strain which is GSH-I deficient such as *E. coli* C 912 may be obtained from colonies without a halo of cysteine-requiring cells. This strain is then treated with a mutagen e.g. NTG in a similar manner to that described above to induce mutation, followed by culturing under the same or similar conditions as described above using a DM medium containing 8-hydroxyquinoline (for example $2 \times 10^{-4}$M) to obtain colonies, from which a mutant strain is obtained which has both GSH-I and GSH-II activities but in which the GSH-1 has a reduced or absent sensitivity to inhibition by glutathione.

The mutant strain thus obtained is hereinafter referred to as a strain having a reduced or absent sensitivity to inhibition by glutathione. A preferred strain of this type is exemplified by *E. coli* RC 912 (FERM-BP No. 47).

(2) Isolation of chromosome DNA from the said strain:
Isolation of chromosome DNA which comprises the genetic information of GSH-I from the feedback insensitive strain and its purification may be effected in conventional manner, for example, by the phenol method [Biochem. Biophys. Acta, Vol. 72, pages 619—629 (1963)].

(3) Integration of the chromosomal DNA into vector DNA:
The thus obtained chromosome DNA is integrated into a vector DNA to obtain a recombinant DNA. The integration may be effected in conventional manner. Thus, for example, the chromosome DNA and vector DNA are cut into pieces by the use of a suitable restriction endonuclease to obtain their fragments which are then mixed together and treated with a DNA ligase. The vector DNA which may be used for the purpose of the present invention includes, for example, pBR 322 plasmid, colicin (Col) El plasmid and Lambda phage, having *Escherichia coli* as the host, and a particularly good result may be obtained by the use of pBR 322 plasmid. Preferred restriction enzymes which may be used include for example Hind III, Eco Ri, Pst I, Bam HI and the like; a preferred restriction enzyme being Hind III. With regard to DNA ligases which may be used for the purpose of the present invention, a DNA ligase originating from $T_4$ phage is preferred.

(4) Introduction of the recombinant DNA into GSH-I deficient strain:
Introduction of the recombinant DNA thus obtained into a GSH-I deficient strain may be effected in conventional manner to obtain a GSH-I deficient strain involving the integrated recombinant DNA, for example, by treatment with calcium ions [Molec. gen. Genet., Vol. 124, pages 1—10 (1973)]. The method of selection of suitable strains involving the recombinant DNA, (i.e. the vector DNA, into which a foreign DNA involving the genetic information of GSH-I is integrated) may vary, with differing conditions, for example, the types of restriction enzyme used for the preparation of the recombinant DNA of interest and the types of vector DNA used. Thus, for example, when Hind III is used as the restriction enzyme and pBR 322 plasmid is used as the vector DNA, the selection may for example be effected in the following manner.

The used strain is cultured by using a DM medium as hereinbefore defined (pH about 7.0; 25—37°C; 16—40 hours) containing tetramethylthiuramdisulfide (40—90 µg/ml) and the resultant colonies are separated, followed by subsequent selection, effected with respect to resistance to ampicillin, sensitivity to tetracycline and the red colouration appearing on application of nitroprusside-ammonia. The final selection is effected by determining the presence or absence of glutathione in the microbial cells and by determining the activity upon GSH-I.

(5) Isolation of recombinant DNA from the strain:
From the strain involving the recombinant DNA, obtained by the above mentioned step, the recombinant DNA may be isolated in conventional manner, for example, by the alkali extraction method [Nucleic Acids Res., Vol. 7, pages 1513—1523 (1979)].

(6) Introduction of the recombinant DNA into a strain having a reduced or absent sensitivity to inhibition by glutathione:
Introduction of the recombinant DNA into a strain having a reduced or absent sensitivity to inhibition by glutathione, as hereinbefore defined i.e. the mutant strain which synthesises both GSH-I and GSH-II but which GSH-I has a reduced or absent sensitivity to inhibition by glutathione, may be effected in conventional manner, for example by treatment with calcium ions [Molec. Gen. Genet., Vol. 124, pages 1—10 (1973)]. The resulting transformant may be obtained with reference to the colonies appearing on a DM medium containing ampicillin. A preferred example of a feedback insensitive strain obtained in this manner is *E. coli* RC 912p (FERM-BP No. 48).

According to a further feature of the present invention there is provided a process for the preparation of glutathione which comprises culturing a microorganism as hereinbefore defined in a medium therefor to accumulate glutathione and recovering the accumulated glutathione therefrom.

By culturing the microorganism of the present invention, a large amount of glutathione is accumulated in the microbial cells. Both synthetic and organic media containing appropriate carbon sources, nitrogen sources and inorganic substances may be used for this purpose. Examples of the carbon sources which may be used for culturing include glucose, sucrose, fructose, starch, starch hydrolyzate and various other hydrocarbons which may be used in an amount of 0.5—5.0%. Examples of the nitrogen sources which may be used include ammonium sulfate, ammonium phosphate, ammonium carbonate, ammonium acetate and various other inorganic and organic compounds containing ammonium; peptone, yeast extract, corn steep liquor, casein hydrolyzate and other inorganic substances containing nitrogen which may be used in an amount of 0.5—2.0%. Various inorganic substances such as potassium dihydrogen phosphate, dipotassium hydrogen phosphate, magnesium sulfate, manganese sulfate and the like may be used as inorganic substances in an amount of 0.005 to 0.5%. The culturing may be effected at a pH of 6—9, preferably 7—8.5 under aerobic conditions with shaking or with shaking and aeration. The culturing temperature is preferably 25—37°C and the culturing time is preferably 16—40 hours to accumulate a large amount of glutathione in the microbial cells.

After completion of the culturing, the glutathione accumulated in the microbial cells may be extracted in conventional manner, for example, with water e.g. by heating to 100°C. The isolation of glutathione from the extracted solution may be effected by any convenient method, for example, by the use of an appropriate ion exchange resin known *per se*.

According to a further feature of the present invention there is provided a process for the preparation of glutathione which comprises culturing a microorganism as hereinbefore defined in a medium therefor to accumulate the enzyme system comprising γ-glutamyl-L-cysteine synthetase and glutathione synthetase, reacting L-glutamic acid, L-cysteine and glycine in the presence of said enzyme system to accumulate glutathione and recovering the glutathione thus obtained.

It is thus possible to prepare glutathione by using the microbial cells and/or a material obtained by treating the microbial cells as an enzyme source if L-glutamic acid, L-cysteine and glycine are reacted in the presence of the enzyme source.

In this case, the expression "a material obtained by treating the microbial cells" denotes, for example, dried microbial cells, cell-free extract obtained by ultrasonic treatment of the microbial cells, enzyme obtained by purifying such a cell-free extracted solution, as well as immobilized microbial cells or immobilized purified enzyme obtained by immobilizing the microbial cells or purified enzyme in conventional manner (e.g. by entrapping with polyacrylamide gel or carrageenan gel).

The concentrations of L-glutamic acid, L-cysteine and glycine in the substrate solution are preferably 5—50 mM, 5—50 mM and 50—100 mM respectively. The reaction may be effected at a pH of 6—9, preferably 7—8.5 and conveniently at a temperature of 20—50°C, preferably 30—37°C.

In order to promote the enzymatic reaction, it is preferred to carry out the reaction in the presence of an adenosine-5'-triphosphate (ATP) regeneration system. In this connection, it is possible to use the reactions effected by various enzymes present in the microorganism used for the process of the present invention such as acetate kinase, enzymes for glycolysis, carbamylphosphate kinase, pyruvate kinase and the like as the ATP regeneration system. For example, the reaction may preferably be effected by using acetate kinase in the presence of magnesium ions e.g. 5—20 mM (in the form of magnesium salts, for example, magnesium sulfate, magnesium chloride and the like), ATP e.g. 2—5 mM and acetylphosphate e.g. 5—10 mM in the substrate solution.

After completion of the reaction, the glutathione accumulated in the reaction mixture may be isolated and purified in conventional manner, for example, by the use of an appropriate ion exchange resin. For example, the pH of the reaction solution may be adjusted to about 3 with sulfuric acid and the solution passed through a cation exchange resin such as Diaion PK-228 H⁺ (commercially available from Mitsubishi Kasei Kogyo K.K., Tokyo) to adsorb glutathione onto the resin, from which glutathione is eluted with about 0.5M ammonium hydroxide. The eluate is adjusted to a pH of about 4.5 with sulfuric acid and passed through an anion exchange resin such as Duolite A2 CH₃COO⁻ form, (commercially available from Diamond Alkali Co., U.S.A.) to adsorb glutathione onto the resin. The adsorbed glutathione is eluted with 0.5M sulfuric acid. 50% ethanol is added to the eluate to give crystals of glutathione, which may then be isolated for example by filtration.

The processes of the present invention are preferably effected by the use of a microorganism of the present invention which is of the species *Escherichia coli* especially *Escherichia coli* RC 912p (FERM-BP No. 48).

The following non-limiting Examples illustrate the present invention.

Example 1
(1) Preparation of a strain of *E. coli* RC 912 having a reduced or absent sensitivity to inhibition by glutathione

*E. coli* B 355 (ATCC 23226), which synthesises -glutamyl-L-cysteine synthetase (E.C. 6.3.2.2., hereinafter referred to as GSH-I) and glutathione synthetase (E.C. 6.3.2.3., hereinafter referred to as GSH-II), is treated to induce a cysteine-requiring strain and a methylglyoxal-resistant strain. The induction of the mutant strains was effected in conventional manner by using N-methyl-N'-nitro-N-nitrosoguanidine (NTG). A portion of the treated strain was then cultured using a minimum medium containing L-cysteine ($2 \times 10^{-5}$M) and having a composition of $K_2HPO_4$ (0.7%), $KH_2PO_4$ (0.3%), $(NH_4)_2SO_4$ (0.1%), $MgSO_4 \cdot 7H_2O$ (0.01%) and glucose (0.5%) [pH about 7.0; hereinafter referred to as DM medium] at a temperature of 37°C for 24 hours with shaking to obtain smaller colonies, from which a cysteine-requiring strain was obtained and cultured pure under the same conditions. Separately, the remaining portion of the strain after induction treatment was cultured under similar conditions by using a DM medium containing methylglyoxal ($2 \times 10^{-3}$M) to

4

obtain larger colonies, from which a methylglyoxal-resistant strain was obtained. The methylglyoxal-resistant strain thus obtained was then transferred to the above-mentioned DM medium containing the cysteine-requiring strain ($10^{-7}$ cells/ml) for culturing under the same conditions to obtain colonies having no halo around them, from which the mutant strain *E. coli* C 912 was obtained. This strain which is deficient in the productivity of GSH-I was then treated with NTG in a similar manner to that described above to induce mutation, followed by culturing under the same conditions as described above by using a DM medium containing 8-hydroxyquinoline ($2\times10^{-4}$M) to obtain colonies, from which a mutant strain was obtained which produced GSH-I and GSH-II but which GSH-I had a reduced or absent sensitivity to inhibition by glutathione, *E. coli* RC 912 (FERM-BP No. 47).

Example 2

(1) Preparation of chromosomal DNA comprising the genetic information of GSH-I (-glutamyl-L-cysteine synthetase):

A mutant strain of *Escherichia coli* designated *E. coli* RC 912 (FERM-BP No. 47) induced from *E. coli* B 355 (as described in Example 1) was cultured at 28°C for 5 hours with shaking by using an L-medium [200 ml; having a composition of peptone (1%), yeast extract (0.5%), glucose (0.1%) and sodium chloride (0.5%); pH 7.2]. The resultant microbial cells were collected and washed with water. The microbial cells were then treated by the method of Saito and Miura [Biochim. Biophys. Acta, *72*, 619—629 (1963)] using phenol to obtain chromosome DNA (900 µg).

(2) Preparation of vector DNA:

pBR 322 plasmid DNA which has genes responsible for the resistance to ampicillin and tetracycline was prepared in the following manner.

*Escherichia coli* B 355 (ATCC 23226), a host of pBR 322 plasmid was cultured at a pH of 7.2 and at a temperature of 37°C by using an L-medium as hereinbefore defined (11). At a later stage of the logarithmic growth phase ($OD_{660}$=about 0.3), chloramphenicol (150 µg/ml) was added to the medium and the culturing was further continued overnight. The microbial cells (3 g wet weight) were collected, washed with water, and then resuspended in 25 ml of 0.1 M Tris-HCl (pH 8.0) containing 10 mM of EDTA. The lysis of the microbial cells was effected by treatment with lysozyme (0.4 mg/ml) and sodium dodecylsulfate (1.4%) at 37°C for one hour. The solution was centrifuged (35,000 r.p.m.; 2 hours) to obtain a supernatant which was treated with 2-butanol to concentrate the DNA. After this, DNA was treated with a ribonuclease (5 µg/ml) at 37°C for 3 hours, followed by CsCl-Ethidium bromide (EtBr) ultracentrifugation (35,000 r.p.m.; 36 hours) to obtain pBR 322 plasmid DNA (450 µg).

(3) Insertion of chromosome DNA fragment into the vector:

The chromosome DNA (1.5 µg) obtained by step (1) was subjected to reaction with a restriction enzyme Hin d III (all of the restriction enzymes are products of Takara Shuzo Co.) (5 unit) at 37°C for 1.5 hours and the vector DNA (1.0 µg) obtained by step (2) was subjected to reaction with the restriction enzyme Hin d III (5 unit) at 37°C for 6 hours to completely cut the chromosome DNA and vector DNA into fragments. The two reaction solutions were each separately heated at 65°C for 10 minutes and then mixed together. The combined solutions were treated with DNA ligase (2 unit, product of Takara Shuzo Co.) orginating from $T_4$ phage at 10°C for 16 hours to effect the insertion. After heating at 65°C for 5 minutes, the reaction solution was treated with ethanol (×2 by volume), allowed to stand at −20°C for one hour and centrifuged (15,000 r.p.m.; 30 minutes) to collect the precipitates which were dissolved in a 5.0 mM tris-HCl buffered solution (pH 7.5; 0.15 ml) to obtain a DNA solution.

(4) Transformation by using plasmid carrying the genetic information of GSH-I:

GSH-I deficient strain *E. coli* C 912 induced from *E. coli* B 355 (ATCC 23226) was cultured at 37°C by using an L-medium as hereinbefore defined (50 ml). At the middle stage of the logarithmic growth phase ($OD_{660}$=about 0.30), the microbial cells were collected, washed twice with a tris-buffer solution (each 20 ml; 50 mM; pH 7.0) containing 50 mM of calcium chloride and was suspended in a similar buffer solution (1.5 ml). To this suspension (0.2 ml) was added the DNA solution obtained by step (3) (0.1 ml). The solution was kept at 0°C for 20 minutes and immediately after this, the solution was heated in the form of a thermal pulse (42°C; 3 minutes) to effect introduction of the DNA into the cells. The suspension was then cultured with shaking at 37°C for 120 minutes using an L-medium as hereinbefore defined (pH 7.2; 3 ml). The microbial cells were collected, washed with water and spread on a DM agar medium [pH 7.0; composed of $KH_2PO_4$ (0.3%), $K_2HPO_4$ (0.7%), $MgSO_4 \cdot 7H_2O$ (0.01%), $(NH_4)_2SO_4$ (0.1%), glucose (0.5%) and agar (1.5%) [20 ml/plate] containing tetramethylthiuramdisulfide (90 µg/ml) for further culturing at 28°C for 24 hours. A transformed strain *E. coli* C 912p was obtained by determining resistance to medicaments such as ampicillin, the sensitivity to tetracycline and the colour reaction using nitroprusside-ammonia of the colonies thus obtained. Finally, the amount of glutathione in the microbial cells and the activity of γ-glutamyl-L-cysteine synthetase (GSH-I) were determined for confirmation of the transformation.

(5) Self-cloning of the plasmid carrying the genetic information of γ-glutamyl-L-cysteine synthetase:

The transformed strain C 912p obtained according to step (4) was cultured using an L-medium as hereinbefore defined [pH 7.2; 100 ml] at 37°C (OD$_{660}$=about 0.3) and treated with chloramphenicol in a similar manner to that described in the above-mentioned step (2). After this, the microbial cells were collected, washed with 0.85% physiological solution of sodium chloride and treated by the method of Birnboim and Doly [Nucleic Acids Res., 7, 1513—1523 (1979)] to prepare a solution of pBR 322 plasmid (hereinafter referred to as pBR 322-gshl) carrying the genetic information for γ-glutamyl-L-cysteine synthetase. The solution was subjected to DNA agarose electrophoresis (agarose 0.7%; 90v; 5 hours) to cut up the band of pBR 322-gshl under irradiation by ultraviolet light. The thus obtained gel was placed in a dialyzing tube and again subjected to electrophoresis under the same conditions to extract DNA from the gel. The extracted solution (5 ml) was treated with 2-butanol to concentrate the DNA and remove the EtBr used for the electrophoresis. Then, ethanol (×2 by volume) was added to the solution to give precipitates of pBR322-gsh I (55 μg) which were dissolved in a tris-HCl buffer solution (5 mM; pH 7.5; 0.5 ml). *Escherichia coli* RC 912 was treated in a similar manner to that described in the above-mentioned step (4) so as to enable the strain to incorporate the DNA and then the above-mentioned solution, pBR322-gsh I solution, was applied to incorporate pBR322-gsh I plasmid into the strain. The strain was then cultured at 37°C for 16 hours using a DM medium as hereinbefore defined (20 ml/plate; pH about 7.0) containing ampicillin (2.5 μg/ml) to form colonies, from which a transformed strain *E. coli* RC 912p (FERM-BP No. 48) was obtained.

Example 3
Strains shown in the following Table 1 were cultured, on each occasion, at 37°C for 16 hours with shaking using a medium (pH 7.0; 2 ml) having the composition of glucose (0.5%), KH$_2$PO$_4$ (0.3%), K$_2$HPO$_4$ (0.7%), MgSO$_4$ · 7H$_2$O (0.01%) and (NH$_4$)$_2$SO$_4$ (0.1%). After completion of the culturing the cultured broth was centrifuged (8,000 r.p.m./10 minutes) to collect the microbial cells which were washed with 0.85% physiological solution of sodium chloride, and glutathione was extracted from the microbial cells with water (1 ml) by heating to 100°C. The amount of glutathione in the extracted solution was measured to determine the glutathione accumulated (by μmole per one gram of the microbial cells).
The results are shown in Table 1.

TABLE 1

| Strain | Accumulated glutathione (μmole/1 g of wet cells |
|---|---|
| (Wild strain) Escherichia coli B 355 | 1.8 |
| (Strain having a reduced or absent sensitivity to inhibition by glutathione) E. coli RC 912 (FERM-BP 47) | 2.8 |
| (Strain of the present invention) E. coli RC 912p (FERM-BP 48) | 7.9 |

Example 4
Strains shown in the following Table 2 were respectively cultured in a similar manner to that described in Example 3. After completion of the culturing, on each occasion, the microbial cells were collected by centrifugation (8,000 r.p.m./10 min.) of the cultured broth and were then subjected to ultrasonic treatment (90 KHz/5 min.) to prepare a cell-free extract. This cell-free extracted solution (0.05 ml) was added to a 50 mM tris buffer solution (9.95 ml; pH 7.5) containing L-glutamic acid (25 mM), L-cysteine (25 mM), glycine (50 mM), magnesium chloride (10 mM), acetylphosphate (10 mM) and ATP (5 mM) to effect the enzymatic reaction at 37°C for one hour. After completion of the reaction, the amount of glutathione formed in the reaction solution was measured to determine the accumulated amount of glutathione per one mg of protein. The results are shown in the following table.

TABLE 2

| Strain | Accumulated amount of glutathione (μmole/1 mg of protein) |
|---|---|
| (Wild strain) Escherichia coli B 355 | 0.11 |
| (Strain having a reduced or absent sensitivity to inhibition by glutathione) E. coli RC 912 (FERM-BP 47) | 0.54 |
| (Strain of the present invention) E. coli RC 912p (FERM-BP 48) | 1.96 |

**Claims**

1. A mutant strain of Escherichia, which synthesises γ-glutamyl-L-cysteine synthetase and glutathionine synthetase required for the synthesis of glutathione from L-glutamic acid, L-cysteine and glycine, the first said enzyme having a reduced or absent sensitivity to inhibition by glutathione and in which the introduction of the chromosomal DNA coding for said enzyme has been effected by transformation with a vector containing the said chromosomal DNA from a strain of E. coli having a reduced or absent sensivitity to inhibition by glutathione.

2. A mutant in claim 1 wherein said vector is an E. coli plasmid.

3. A mutant as claimed in claim 2, in which the plasmid is pBR 322 plasmid.

4. A mutant as claimed in claim 1, in which the strain is Escherichia coli.

5. A mutant as claimed in claim 1, which is Escherichia coli RC 912p (FERM-BP 48).

6. A mutant as claimed in claim 1, 2 or 3 wherein the strain which is transformed is Esherichia coli RC 912 (FERM-BP 47).

7. A mutant as claimed in claim 6 in which the strain having a reduced or absent sensitivity to inhibition by glutathione is also Escherichia coli RC 912 (FERM-BP 47).

8. A process for the preparation of glutathione, which comprises culturing a mutant strain as defined in any of claims 1 to 7 in a medium to accumulate glutathione in the cultured broth and recovering glutathione therefrom.

9. A process as claimed in claim 8, in which the recovery is effected by extracting glutathione with water from the microbial cells.

10. A process for the preparation of glutathione, which comprises culturing a strain as defined in any of claims 1 to 7 in a medium to accumulate the enzyme system as defined in claim 1 and placing the thus-obtained enzyme system into contact with a substrate solution of L-glutamic acid, L-cysteine and glycine to effect an enzymatic reaction for the formation of glutathione and recovering glutathione from the reaction solution.

11. An E. coli plasmid containing the DNA coding for a γ-glutamyl-L-cysteine synthetase which has reduced or absent sensitivity to inhibition by glutathione.

12. An E. coli plasmid as claimed in claim 11 containing the DNA coding for the γ-glutamyl-L-cysteine synthetase present in Escherichia coli RC 912 (FERM-BP 47).

**Patentansprüche**

1. Mutantenstamm von Escherichia, welcher γ-Glutamyl-L-cystein Synthetase und Glutathion Synthetase synthetisiert, die zur Synthese von Glutathion aus L-Glutaminsäure, L-Cystein und Glycin erforderlich sind, wobei das erstgenannte Enzym eine verminderte oder fehlende Sensitivität gegenüber einer Inhibition durch Glutathion aufweist, und in welchen die Einführung der codierenden chromosomalen DNA für dieses Enzym durch Transformation mit einem Vektor erfolgt, der diese chromosomale DNA aus einem E. coli Stamm enthält, der eine verminderte oder fehlende Sensitivität gegenüber einer Inhibition durch Glutathion aufweist.

2. Mutante nach Anspruch 1, worin der Vektor ein E. coli Plasmid ist.

3. Mutante nach Anspruch 2, worin das Plasmid ein pBR 322 Plasmid ist.

4. Mutante nach Anspruch 1, worin der Stamm Escherichia coli ist.

5. Mutante nach Anspruch 1, mit der Bezeichnung Escherichia coli RC 912p (FERM-BP 48).

6. Mutante nach Anspruch 1, 2 oder 3, worin der transformierte Stamm Escherichia coli RC 912 (FERM-BP 47) ist.

7. Mutante nach Anspruch 6, worin der Stamm mit verminderter oder fehlender Sensitivität gegenüber Inhibition durch Glutathion Escherichia coli RC 912 (FERM-BP 47) ist.

8. Verfahren zur Herstellung von Glutathion, umfassend die Kultivierung eines Mutantenstammes gemäß einem der Ansprüche 1—7 in einem Medium zur Anreichung von Glutathion im Kulturmedium und die Isolierung des Glutathions.

9. Verfahren nach Anspruch 8, worin die Isolierung durch Extraktion des Glutathions aus den Zellen der Mikroorganismen mit Wasser erfolgt.

10. Verfahren zur Herstellung von Glutathion, umfassend die Kultivierung eines Stammes nach einem der Ansprüche 1—7 in einem Medium zur Anreicherung des Enzymsystems gemäß Anspruch 1 und das in Kontakt bringen des so erhaltenen Enzymsystems mit einer Substratlösung aus L-Glutaminsäure, L-Cystein und Glycin, wobei eine enzymatische Reaktion zur Bildung von Glutathion erfolgt, und die Isolierung des Glutathions aus der Reaktionslösung.

11. Ein *E. coli* Plasmid, welches die codierende DNA für eine γ-Glutamyl-L-cystein Synthetase enthält, die eine verminderte oder fehlende Sensitivität gegenüber einer Inhibition durch Glutathion aufweist.

12. Ein *E. coli* Plasmid gemäß Anspruch 11, welches die codierende DNA für die γ-Glutamyl-L-cystein Synthetase in *Escherichia coli* RC 912 (FERM-BP 47) enthält.

## Revendications

1. Souche mutante de *Escherichia,* qui synthétise de la 5γ-glutamyl-L-cystéine-synthétase et de la glutathion-synthétase nécessaires à la synthèse du glutathion à partir d'acide L-glutamique, de L-cystéine et de glycine, le premier de ces enzymes ayant une sensibilité réduite ou nulle à l'inhibition par le glutathion, et dans laquelle l'introduction de l'ADN chromosomique codant pour ledit enzyme a été effectuée par transformation avec un vecteur contenant ledit ADN chromosomique provenant d'une souche de *E. coli* ayant une sensibilité réduite ou nulle à l'inhibition par le glutathion.

2. Mutant suivant la revendication 1, dans lequel le vecteur est un plasmide de *E. coli*.

3. Mutant suivant la revendication 2, dans lequel le plasmide est le plasmide pBR 322.

4. Mutant suivant la revendication 1, dans lequel la souche est une souche de *Escherichia coli*.

5. Mutant suivant la revendication 1, qui est *Escherichia coli* RC 912p (FERM-BP 48).

6. Mutant suivant la revendication 1, 2 ou 3, dans lequel la souche qui est transformée est *Escherichia coli* RC 912 (FERM-BP 47).

7. Mutant suivant la revendication 6, dans lequel la souche ayant une sensibilité réduite ou nulle à l'inhibition par le glutathion est également *Escherichia coli* RC 912 (FERM-BP 47).

8. Procédé de préparation de glutathion, qui consiste à cultiver une souche mutante suivante l'une quelconque des revendications 1 à 7 dans un milieu pour accumuler le glutathion dans le bouillon de culture et à séparer le glutathion de ce bouillon.

9. Procédé suivant la revendication 8, dans lequel la séparation est effecutée en extrayant le glutathion des cellules microbiennes avec de l'eau.

10. Procédé de préparation de glutathion, qui consiste à cultiver une souche suivant l'une quelconque des revendications 1 à 7 dans un milieu pour accumuler le système enzymatique défini suivant la revendication 1, et à placer le système enzymatique ainsi obtenu en contact avec une solution de substrat constituée d'acide L-glutamique, de L-cystéine et de glycine pour effectuer une réaction enzymatique destinée à la formation de glutathion, et à séparer le glutathion de la solution réactionnelle.

11. Plasmide de *E. coli* contenant l'ADN codant pour une γ-glutamyl-L-cystéine-synthétase qui possède une sensibilité réduite ou nulle à l'inhibition par le glutathion.

12. Plasmide de *E. coli* suivant la revendication 11, contenant l'ADN codant pour la γ-glutamyl-L-cystéine-synthétase présente dans *Escherichia coli* RC 912 (FERM-BP 47).